# FASCICULE DE BREVET EUROPEEN

(11) **EP 2 241 290 B1**
(45) Date de publication et mention de la délivrance du brevet: **09.05.2012**
(21) Numéro de dépôt: 10007388.1
(22) Date de dépôt: 14.03.2008
(51) Int. Cl.: A61F 2/42, A61F 2/00

(54) **Dispositif d'osteosynthèse**
Osteosynthetische Vorrichtung
Osteosynthetic device

(30) Priorité: 20.03.2007 FR 0702003
(43) Date de publication de la demande: 20.10.2010
(62) Demande divisionnaire de: 08799857.1
(73) Titulaire: Memometal Technologies, société anonyme, 35170 Bruz (FR)
(72) Inventeur: Prandi, Bernard, 35700 Rennes (FR); Bellemere, Philippe, 44000 Nantes (FR); Augoyard, Marc, 69160 Tassin la demi Lune (FR); Peyrot, Jacques, 69160 Tassin la demi Lune (FR); Meusnier, Tristan, 42000 Saint Etienne (FR)
(74) Mandataire: Benech, Frédéric

(56) Documents cités:
- WO-A-2006/109004
- FR-A- 2 846 545
- US-A- 5 425 777

## Description

L'invention se rattache au secteur technique des implants orthopédiques, notamment pour arthrodèses et ostéosynthèses.

On rappelle qu'un implant d'ostéosynthèse doit servir à maintenir en place deux (ou plus) parties d'un même os fracturé ou coupé par une opération de chirurgie (ostéotomie), le temps nécessaire à la consolidation de cet os (typiquement 3 mois).

On rappelle qu'une arthrodèse est le blocage d'une articulation par voie chirurgicale pour souder deux os en un seul, au moyen d'un dispositif d'ostéosynthèse.

On rappelle que le but de toute ostéosynthèse et particulièrement dans le cas d'une arthrodèse est d'obtenir une très bonne stabilité aussi bien primaire que secondaire afin d'obtenir la consolidation dans les meilleures conditions, c'est-à-dire dans la position choisie par le chirurgien, en minimisant les problèmes de douleur postopératoire et les oedèmes, et en raccourcissant autant que faire se peut le temps de consolidation.

Pour ce résultat, la stabilité de l'ostéosynthèse liée à l'implant est critique. De plus, l'implant doit également apporter et maintenir une légère compression sur les parties d'os à fusionner, ce qui facilite cette consolidation.

Différentes solutions techniques ont été proposées, pour réaliser une arthrodèse, notamment au niveau des extrémités (pied, main, poignet, cheville, ...).

On peut citer, par exemple, des agrafes basiques qui n'assurent pas un bon maintien pendant la consolidation, et des agrafes à mémoire de forme qui permettent de mettre en compression les deux parties d'os à consolider, ce qui correspond au but recherché.
Toutefois, pour obtenir une stabilité satisfaisante, il est nécessaire de mettre deux, voire trois agrafes, dans des plans différents. Il en résulte un encombrement important, ce qui limite les applications, notamment sur des petits os (par exemple au niveau des doigts ou des orteils).

Il est également courant d'utiliser des plaques et vis extramédullaires ou extra osseuses, qui nécessitent également un encombrement relativement important et ne peuvent être utilisées sur les phalanges terminales des doigts (arthrodèse interphalangienne distale par exemple). Par ailleurs la stabilité à moyen terme de ces systèmes n'est pas toujours au rendez-vous (débricolage du montage).

Certains types de vis peuvent être utilisés en intramédullaire, mais dans ce cas, la voie d'abord nécessite un abord pulpaire ce qui peut générer des complications graves (sepsis, ...) et un inconfort pour le patient.

On peut également utiliser des broches qui présentent un encombrement réduit. Toutefois la stabilité obtenue n'est pas satisfaisante (problèmes de migration) et il est généralement nécessaire de procéder au retrait après consolidation. De plus avec de tels dispositifs, le patient ne peut immerger le doigt ou l'orteil traité puisque la broche est en général débouchante à l'extérieur de la peau.

Il existe pour des os longs (tibia, fémur, humérus, ....) des systèmes d'ostéosynthèse intramédullaires. On connaît par exemple les clous centromédullaires vérouillables. Outre que la technique de vérouillage est difficile, ils ne peuvent être miniaturisés pour la chirurgie des extrémités (main et pied).

Enfin il existe des dispositifs intramédullaires à mémoire de forme permettant de résoudre une partie de la problématique de l'arthrodèse ou de l'ostéosynthèse des petits fragments : par exemple les solutions décrites dans le brevet 2 846 545, le brevet 2 884 406 ou WO 2006/109004.

Le brevet 2 846 545 décrit un dispositif en H qui s'ouvre dans le corps en forme de X, grâce à l'utilisation de la mémoire de forme réglée vers 37°C, chaque patte étant implantée dans un trou calibré.

En pratique un tel système ne permet pas une introduction correcte dans l'os. En effet, la réalisation de 2 trous parallèles est très difficile dans une phalange du fait de la taille et surtout les pattes parallèles ont tendance à s'ouvrir naturellement lors de l'introduction et ont plutôt ce faisant un effet de distraction des deux fragments que de mise en compression.

Par ailleurs, l'utilisation de la mémoire de forme est très limitative par les contraintes qu'elle génère pour les chirurgiens, notamment de gestion de la température : il faut impacter l'implant dans l'os dans leur phase froide fermée avant qu'ils s'ouvrent à la température du bloc. Ceci nécessite de mettre l'implant dans un support, de le stocker au froid et d'aller très vite pour l'implanter.

Enfin les pattes étant droites conduisent dans leur forme ouverte à créer un appui ponctuel à leur extrémité qui n'apporte pas une bonne tenue et peut abîmer l'os.

Le brevet 2 884 406 présente un système qui permet une introduction facilité soit par la forme (oeil), soit par un support ou une pince qui maintient fermées les pattes de l'implant pendant l'introduction.

Néanmoins, ces systèmes ne fonctionnent pas de manière très fiables, car ils ne définissent pas les critères optimaux permettant une bonne introduction dans l'os et un bon ancrage : les zones d'ancrage ont toujours tendance à s'ouvrir trop tôt, ce qui bloque l'introduction

L'invention s'est fixée pour but de remédier à tous ces inconvénients d'une manière simple, fiable, efficace et rationnelle.

Le problème que se propose de résoudre l'invention est de définir les critères de réussite pour un implant intramédullaire, facile à poser et efficace pour générer une stabilité primaire et secondaire du foyer d'ostéosynthèse ou d'arthrodèse grâce à sa rigidité et à sa composante de compression.

### Invention

L'implant de l'invention est caractérisé par le fait qu'il comprend deux zones d'ancrage osseux de part et d'autre d'une zone rigide de stabilité, résistante au cisaillement, ces deux zones d'ancrage présentant à leur base une possibilité de déformation important (notamment par élasticité) et un design tels qu'elles peuvent adopter une position fermée (notamment grâce à une pince adaptée serrée à leur base) pour une introduction facile dans un trou centromédullaire calibré (réalisé avec un instrument adéquat), et qu'elles présentent dans le site osseux de part cette configuration particulière la possibilité de réaliser l'impaction finale sans effet distracteur sur l'os et présente une expansion suffisante pour assurer une bonne accroche dans l'os.

Les zones d'ancrage peuvent se déformer à leur base par élasticité, superélasticité ou mémoire de forme et sont typiquement constituées par des branches ou pattes, éventuellement reliées (forme en olive ou ballon de rugby). Ces branches présentent dans la forme ouverte un angle positif vers l'extérieur à leur base et sont courbées vers l'intérieur vers leur extrémité tandis quand forme fermée, l'angle à la base est inversé, c'est-à-dire négatif ou tourné vers l'intérieur ce qui permet d'avoir la largeur au niveau des extrémités (côté impaction) plus fine que leur base afin de ne pas risquer de s'impacter dans l'os et de bloquer la pénétration de l'implant.

L'invention trouve une application particulièrement avantageuse, qui ne saurait toutefois être considérée comme limitative, pour la réalisation des arthrodèses au niveau des phalanges des doigts et des orteils, notamment pour les articulations interphalangiennes proximales et distales dans la main et le pied.

L'ensemble est implantable par voie dorsale (ou éventuellement latérales ou palmaire/plantaire), mais sans abord pulpaire, ce qui minimise les risques d'infection et améliore le confort patient.

Pour tenir compte des caractéristiques anatomiques, les zones d'ancrages sont reliées à la zone médiane servant de résistance (notamment au cisaillement) au niveau du foyer d'ostéosynthèse par des zones de liaisons plus ou moins longues, et la zone centrale peut présenter une angulation pour s'adapter aux caractéristiques de l'arthrodèse recherchée.

Le matériau constitutif de l'implant objet de l'invention doit permettre une certaine ouverture minimale des zones d'ancrage une fois l'implant dans le corps. Il peut donc s'agir de n'importe quel matériau implantable suffisamment élastique tel que de l'acier inoxydable, du titane, un matériau biorésorbable tel que l'acide polylactide PLLA ...
Préférentiellement, l'implant objet de l'invention est réalisé à partir d'un matériau à mémoire de forme utilisé pour sa propriété de superélasticité (ou élasticité liée à la transformation de phase austénite martensite sous contrainte) qui présente la plus grande plage d'élasticité connue (jusqu'à 8% en allongement élastique équivalent en traction pour un implant en Nitinol, alliage de Nickel - Titane comprenant environ 55,5 à 56% en poids de Nickel, le reste en titane).

L'utilisation d'un matériau présentant une mémoire de forme thermique vers 37°C est également possible.

L'invention est exposée ci-après plus en détail à l'aide des figures et dessins annexés dans lesquels :
- La figure 1 présente un exemple d'implant selon l'invention dans sa position totalement ouverte, en 3 dimensions.
- La figure 2 présente ce même exemple dans sa position fermée d'introduction, dans son plan principal,
- La figure 3 présente un exemple d'implant selon l'invention dans sa position totalement ouverte, dans son plan principal.
- La figure 4 présente un exemple d'implant coudé selon l'invention dans sa position totalement ouverte
- La figure 5 (5a à 5f) présente la séquence d'implantation : fermé, introduit à moitié toujours contraint, introduit complètement d'un côté (libre dans l'os), et la même séquence de l'autre côté.

### Description des caractéristiques de l'invention

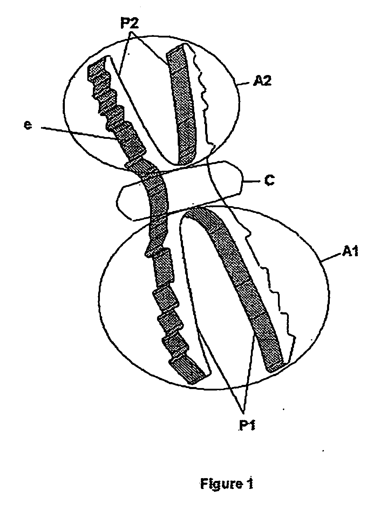

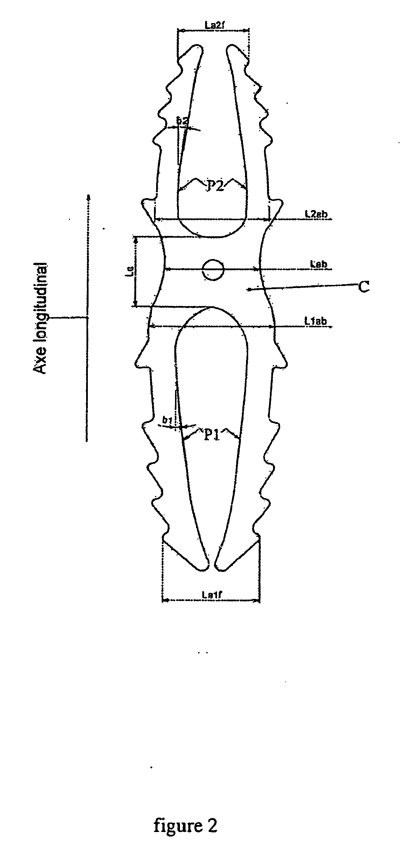

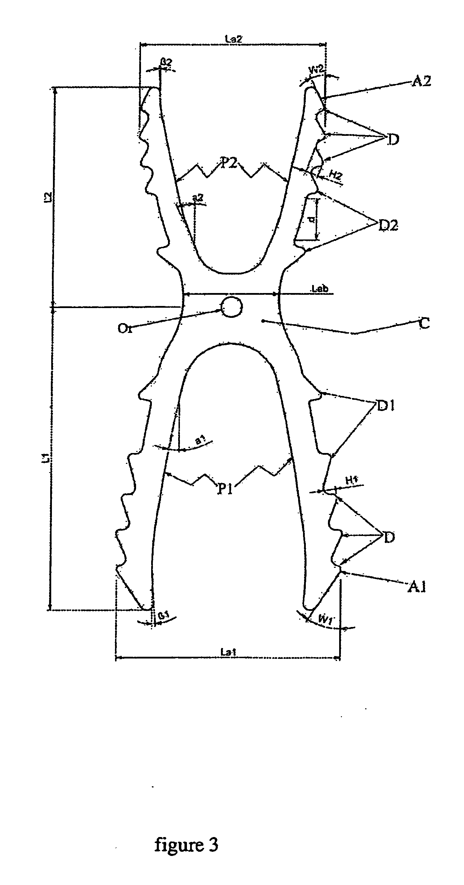

L'implant se présente sous la forme de 2 zones d'ancrages (A1) et (A2) reliées par une zone centrale (C) (figure 1) et éventuellement des zones de liaison intermédiaires telles que en position fermée, la forme soit sensiblement inscrite dans un rectangle très allongé (figure 2), et en forme ouverte corresponde plus à une forme en X plus large du fait de l'ouverture des zones d'ancrage (figure 3).

Les zones d'ancrages (A1), respectivement (A2) sont réalisées en général chacune par 2 pattes (P1), respectivement (P2) de longueur (L1), respectivement (L2) (figure 3).

La section transverse de l'implant est adaptée aux sites d'implantation, mais préférentiellement méplate afin d'avoir une bonne résistance mécanique et un encombrement réduit (typiquement l'épaisseur (e) est de l'ordre de 1 à 2 mm) (figure 1).

La figure 2 décrit la position fermée avec les différentes largeurs de l'implant : (Lab) est la largeur de la zone centrale (C), (L1ab) et (L2ab) sont les largeurs à la base des zones d'ancrage respectivement (A1) et (A2). Ces 3 largeurs peuvent être égales ou légèrement différentes pour s'adapter au site osseux. Typiquement les largeurs sont de l'ordre de 2 à 5 fois l'épaisseur (soit 2 à 10 mm). Ces dimensions sont bien adaptées aux différentes indications de la main et du pied mais ne sont pas limitatives puisqu'elles dépendent du site osseux du patient opéré.

Les zones d'ancrages (A1), (A2) sont aptes à s'écarter par effet élastique ou par effet mémoire de forme à leur base, afin que la largeur maximale en position ouverte aux extrémités (La1) et (La2) (figure 3) soit au moins égale à la largeur de la base de la même zone d'ancrage en position fermée augmentée de 50% minimum, ou augmentée de 1,5 mm minimum. C'est-à-dire que La1 > L1ab + 50% ou La1 > L1ab + 1,5 mm et que La2 > L2ab + 50% ou La2 > L2ab + 1,5 mm.
Ce critère d'ouverture est nécessaire afin d'avoir suffisamment de tenue dans l'os.

Ainsi que le décrit la figure 3, les pattes (P1), (P2) sont sensiblement droites à leur base (sur environ 1/3 à la moitié de leur longueur) puis de formes arrondies vers l'intérieur à leur extrémité (sur environ 1/3 à la moitié de leur longueur). En position ouverte la partie droite des pattes (P1), respectivement (P2) présente un angle (a1), respectivement (a2) avec l'axe de l'implant tourné vers l'extérieur (figure 3), positif, tandis qu'en position fermée, cet angle devient l'angle (b1), respectivement (b2) négatif tourné vers l'intérieur (figure 2). La partie supérieure (vers l'extrémité) des pattes ne subit quasiment pas de déformation particulière entre les 2 formes ouverte et fermée.
Cette disposition géométrique particulière permet qu'en position fermée les pattes se touchent quasiment à l'extrémité (figure 2), et que la largeur au niveau de l'extrémité en position fermée (La1f), respectivement (La2f) soit inférieure à la largeur de la base (L1ab), respectivement (L2ab), ce qui permet une introduction facile sans distraction du fragment osseux distal et permet également d'obtenir le mouvement ouvert/fermée par une déformation localisée à la base des pattes, c'est-à-dire en laissant libre la zone distale pour pouvoir introduire cette zone dans l'os.

Afin d'obtenir à la fois une introduction aisée et un mouvement suffisant d'ouverture, les angles (a1), (a2) sont préférentiellement compris entre +5° et +25° et les angles (b1), (b2) entre 0° et -15°.

Préférentiellement la largeur des extrémités des zones d'ancrage en position fermée (La1f), respectivement (La2f) est plus faible que la largeur de la base desdites zones (L1ab), respectivement (L2ab) diminuée de 20% : Lalf< Llab-20% et La2f< L2ab-20%.

Les pattes ou les zones d'ancrage sont ainsi « articulées » à leur base, et peuvent donc être maintenues en position fermées par un support ou mieux une pince, positionnée, à un endroit adéquat défini notamment dans le cas d'un matériau élastique (pour un matériau à mémoire de forme, cela n'est pas forcément nécessaire puisque la forme n'évolue pas tant que la température d'activation n'est pas atteinte), cette pince ne couvrant pas plus de la moitié de la longueur des pattes, ce qui permet une introduction de l'implant d'au moins la moitié dans son logement.

La tangente côté interne en extrémité des pattes (P1), respectivement (P2) en position ouverte fait un angle (β1), respectivement (β2) avec l'axe longitudinal de l'implant proche de 0°, afin d'avoir une bonne surface de contact osseux sur toute la longueur de la patte en position ouverte et éviter que seules les extrémités touchent l'os (figure 3).

Dans le site d'implantation, à la température du corps, l'implant peut encore être en position fermée, ou pattes parallèles ou semi-ouvert, afin que l'effort exercé par l'ouverture des pattes se transmette au niveau de l'os et assure un bon ancrage.

Cette disposition « en olive » des pattes, associée à une « articulation » de la base et associée à une introduction minimale de la moitié de leur longueur permet de finir l'impaction, une fois la pince enlevée.

Afin de garantir un bon fonctionnement, l'élasticité ou la mémoire de la pièce doit permettre de passer de la forme fermée (typiquement largeur 2 à 4 mm selon la taille du site) à une forme ouverte avec un mouvement significatif (+1,5 à + 3 mm environ).
De même la force d'expansion des pattes (ou de gonflement de l'olive) doit être significative : typiquement 1 à 3 kg pour une arthrodèse des extrémités (force mesurée à 37°C en position d'introduction bloquée), sans être excessive : il importe que les pattes ne puissent pas s'ouvrir totalement et que l'os résiste afin d'avoir réellement une force de maintien.

Les pattes (P1), (P2) ou ailettes peuvent présenter une surface rugueuse ou mieux des crans (D) (figure 3) sur leurs surfaces externes destinées à s'impacter dans l'os spongieux et à exercer un bon ancrage. La hauteur typique de ces crans (H1), (H2) est de l'ordre de 0,5 mm.
L'ouverture des pattes doit être au minimum de 1,5 fois cette hauteur afin d'assurer une impaction réelle des crans dans l'os, soit 1,5 mm.

Les pattes (P1), (P2) peuvent également présenter une surface recouverte d'un revêtement ostéointégrateur telle que l'hydroxyhapatite (HAP) destinée à faciliter l'ancrage.

Afin de faciliter l'introduction dans l'os, les extrémités des pattes (P1), resp. (P2) sont biseautées avec un angle vers l'intérieur par rapport à l'axe longitudinal de l'implant (W1), resp. (W2) (figure 3). Cet angle est typiquement compris entre -20° et -40°.

Par essai sur cadavres frais, et expérience, il a été déterminé un niveau optimal de la force avec un minimum permettant d'ancrer les crans dans l'os spongieux et une force maximale pour être sur de ne pas détériorer le site d'implantation

Après tests et expérience, il a été trouvé une zone idéale de maximum 20% de la limite élastique de l'os mesurée en forme fermée bloquée à 37°C, ce qui compte tenu des dimensions de l'implant conduit à des valeurs maximales de l'ordre de 3 kg, et la nécessité d'un abaissement rapide dès que l'ancrage est obtenu, soit une force divisée par 2 en position semi-ouverte (une force de 0,5 à 1,5 kg permet un bon maintien).
En effet si la force d'ouverture est supérieur à environ 3 kg, l'introduction dans l'os devient beaucoup plus difficile, voir impossible dès 4 kg.
Enfin afin de garantir une non détérioration du site, il est nécessaire que la force devienne négligeable pour une ouverture quasi-complète.
Ces valeurs sont indicatives et dépendent du site d'arthrodèse et de la qualité osseuse.

Sur une version de l'invention, des crans (D1), resp.(D2) côté externe aux pattes (P1), resp. (P2) permettent de positionner une pince de serrage et d'introduction à la base des pattes (P1), (P2) (figure 3). Ces crans sont symétriques par paire de pattes et leur écartement (d) est le même sur les pattes (P1) que sur les pattes (P2).

La zone centrale (C) doit avoir une longueur minimale (Lc) égale à la longueur (d) entre les crans (D1), respectivement (D2), afin que même en cas de mouvement de l'implant lors de l'impaction finale cette zone (C) reste dans le foyer d'arthrodèse et assure sa fonction de résistance.
Dans une version de l'invention, il est prévu un orifice (Or) dans cette zone centrale permettant de positionner une broche de maintien pour éviter une éventuelle migration de l'implant au moment de l'impaction finale.

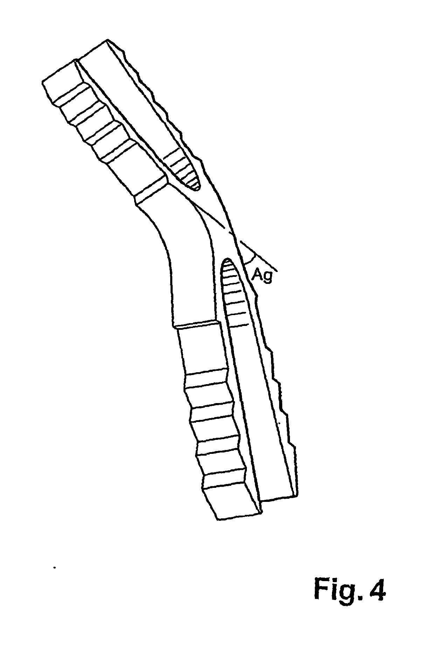

Ainsi que le montre la figure 4, cette zone centrale peut être angulée d'un angle (Ag) défini entre les 2 plans principaux formés par les pattes (P1) d'une part et (P2) d'autre part pour s'adapter aux contraintes chirurgicales de réglage de la position de l'arthrodèse. Dans la majorité des cas, l'angle (Ag) est fixé entre 0° (position plate typiquement pour un index) et 30°(typiquement pour un 5' doigt) .

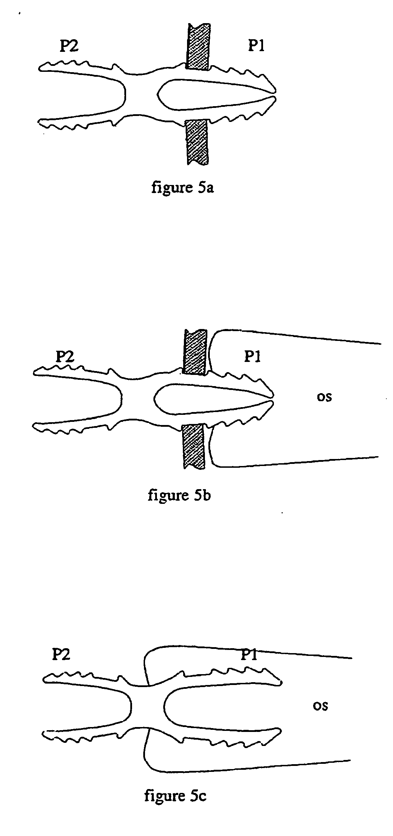

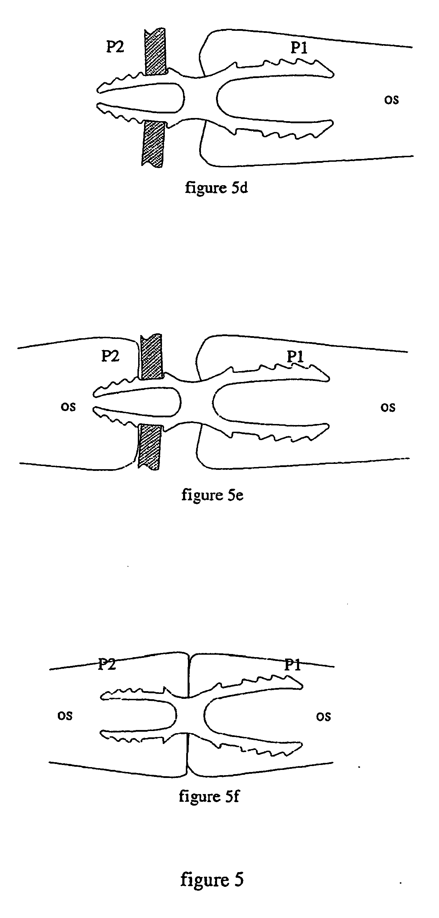

A titre d'exemple une technique opératoire de l'implantation du dispositif de l'invention pour le cas d'un implant élastique ou superélastique est la suivante telle que décrit sur la figure 5 :
Abord par voie dorsale
Résection des cartilages et ostéophytes
Perçage centromédullaire à l'aide d'un instrument adapté permettant de faire un trou calibré rectangle de largeur sensiblement (L1ab) ou (L2ab) et d'épaisseur sensiblement e (râpe adaptée)
Fermeture à la pince côté P1 (figure 5a)
Introduction implant côté P1 à la moitié minimum (figure 5b)
Enlèvement pince
Introduction totale côté P1 (figure 5c)
Fermeture à la pince côté P2 (figure 5d)
Mise en place de l'os côté P2 sur l'implant côté P2 à la moitié environ (figure 5^{e})
Enlèvement de la pince
Impaction manuelle de l'os côté P2 sur l'os P1 (figure 5f)

### Description détaillée des caractéristiques de l'invention et exemples de réalisation

Dans une forme de réalisation particulière, destinée à l'arthrodèse de l'InterPhalangienne Distale (main), l'implant est réalisé dans un alliage nitinol superélastique (nickel-titane dans la proportion 55,8% poids Nickel et 44,2% titane)

La section de la zone centrale (C) est de Lab x e = 2,8 x 1,2 mm et les pattes sont dissymétriques pour s'adapter au mieux aux formes de l'os, minimiser la section métallique implantée et permettre une expansion suffisante pour un bon ancrage. La longueur des pattes est de L2=6,5 mm en distale (P2) et L1=9 mm en proximale (P1)
La longueur de la zone centrale (C) est de 3 mm, ce qui autorise un petit décalage lors de la fermeture, sans nuire à la résistance au cisaillement.
Pour s'adapter au choix du chirurgien, cette zone centrale peut être coudée (typiquement plate ou 15° ou 25°)

En position fermée, la largeur de la base proximale L1ab de 3,8 mm et de la base distale L2ab est de 3,0 mm.
L'ouverture des pattes (P1), respectivement (P2) est de 2,5 mm, respectivement 2,2 mm, c'est-à-dire que La1 vaut 6,3 mm et La2 vaut 5,2 mm.
En position ouverte l'angle à la base des pattes est de a1 =10° et a2=22°
La portion droite est d'environ 45% de la longueur totale
La courbure de l'extrémité distale des pattes est calculée pour que l'angle de la tangente à l'extrémité soit de β1= -5° et β2= -3°
En position fermée, l'angle à la base des pattes est b1=-4°, b2= -2°
Et la largeur à l'extrémité est La1f = 2,5 mm et La2f = 2,1 mm

Dans une forme de réalisation de l'invention, des crans de hauteur 0,5 mm sont réparties sur les pattes (1 cran tous les 0,8 mm environ)

L'angle d'incidence de l'extrémité des pattes (crans compris) est de w1 = 33° et w2 = 24°, ce qui permet une introduction aisée sans effet distracteur entre les 2 morceaux d'os à ostéosynthéser

Le dessin arrondi des zones d'ancrage permet d'obtenir en forme ouverte une surface de contact maximisée sur toute la longueur, avec un effet d'impaction dans l'os spongieux, et donc un effet de tassement du spongieux.

Dans un autre exemple, plus adapté à une arthrodèse au niveau du pouce, les dimensions sont plutôt les suivantes :
Largeurs fermées : L1ab = 6,5 mm, L2ab = 5 mm, avec une ouverture de 3 à 4mm environ pour obtenir : La1 = 11 mm et La2 = 8 mm et L1=13 mm et L2=9mm

## Revendications

1. Dispositif d'ostéosynthèse intra médullaire de deux parties d'os, notamment de la main ou du pied, constitué d'un corps de section principale méplate, comprenant deux zones (A1, A2) d'ancrage osseux de part et d'autre d'une zone centrale (C) rigide de stabilité, résistante au cisaillement, ces deux zones étant chacune respectivement formée par deux pattes (P1, P2) symétriques autour d'un axe longitudinal du dispositif, déformables entre une forme fermée pour introduction dans un trou osseux précalibré, et une forme ouverte pour accrochage dans l'os,
**caractérisé en ce que** les pattes présentent dans leur forme ouverte un angle (a1, a2) à la base positif tourné vers l'extérieur de leur base, et dans leur forme fermée un angle (b1, b2) à la base négatif, la largeur au niveau des extrémités en position fermée (La1f, La2f) étant inférieure à la largeur au niveau de la base (L1ab, L2ab).

2. Dispositif selon la revendication 1, **caractérisé en ce que** les pattes (P1, P2) sont sensiblement droites à leur base puis de forme arrondie vers l'intérieur à leur extrémité.

3. Dispositif selon la revendication 2, **caractérisé en ce que** les pattes (P1, P2) sont sensiblement droites sur 1/3 à la moitié de leur longueur à leur base et de forme arrondie sur 1/3 à la moitié de leur longueur à leur extrémité.

4. Dispositif selon l'une quelconque des revendications précédentes **caractérisé en ce que** les extrémités des pattes sont propres à se toucher quasiment en forme fermée.

5. Dispositif selon l'une quelconque des revendications précédentes, **caractérisé en ce que** il est réalisé dans un matériau implantable à mémoire de forme présentant une propriété d'élasticité ou de super-élasticité mécanique.

6. Dispositif selon l'une quelconque des revendications 1 à 4, **caractérisé en ce que** il est réalisé dans un matériau implantable à mémoire de forme thermique.

7. Dispositif selon l'une quelconque des revendications précédentes, **caractérisé en ce que** en forme ouverte les pattes (P1, P2) sont agencées pour former à leur base un angle (a1, a2) par rapport à l'axe du dispositif vers l'extérieur compris entre 5° et 25°, et sont recourbées vers l'intérieur vers leur extrémité pour que la tangente coté interne à l'extrémité forme un angle avec l'axe longitudinal voisin de 0°.

8. Dispositif selon l'une quelconque des revendications précédentes, **caractérisé en ce que** en forme fermée les pattes (P1, P2) forment à leur base un angle (b1, b2) vers l'intérieur compris entre - 0° et - 15°, sans modification notable sur le dernier tiers de leur longueur, pour que la largeur extérieure aux extrémités en forme fermée soit inférieure à la largeur de la base.

9. Dispositif selon l'une quelconque des revendications précédentes, **caractérisé en ce que** les bases des zones (A1, A2) d'ancrages sont déformables, pour le passage des pattes (P1, P2) de la forme fermée à la forme ouverte.

10. Dispositif selon l'une quelconque des revendications précédentes, **caractérisé en ce que** la largeur aux extrémités (La1, La2) de la forme ouverte est supérieure ou égale à la largeur aux extrémités (La1f, La2f) de la forme fermée de 50% ou de 1,5 mm.

11. Dispositif selon l'une quelconque des revendications précédentes, **caractérisé en ce que** la largeur (La1f, La2f) aux extrémités de la forme fermée est inférieure à la largeur de la base (L1ab, L2ab) de 20%.

12. Dispositif selon l'une quelconque des revendications précédentes, **caractérisé en ce que** les extrémités sont biseautées.

13. Dispositif selon l'une quelconque des revendications précédentes, **caractérisé en ce que** les zones (A1, A2) d'ancrage présentent des crans (D) sur la face externe des pattes ou une surface rugueuse.

14. Dispositif selon l'une quelconque des revendications précédentes, **caractérisé en ce que** il comporte dans la moitié inférieure des zones (A1, A2) d'ancrage, du côté de la base, une zone ou un logement de longueur déterminée délimité par des crans (D1, D2), de positionnement d'un instrument de fermeture et maintien en position fermée.

15. Dispositif selon la revendication 14, **caractérisé en ce que** la zone centrale (C) à une longueur minimale (Lc) égale à la longueur (d) de la zone ou logement entre les crans (D1, D2).

16. Dispositif selon l'une quelconque des revendications précédentes, **caractérisé en ce que** la zone centrale (C) est coudée et présente un angle (Ag) entre les deux zones d'ancrage pouvant atteindre 30°.

17. Dispositif selon l'une quelconque des revendications précédentes, **caractérisé en ce que** la force d'expansion des pattes (P1, P2) entre la forme ouverte et la forme fermée est comprise entre 1 et 3 Kg.

## Claims

1. Device for intramedullary osteosynthesis of two bone portions, in particular in the hand or foot, consisting of a body having a flat main cross-section, including two bone-anchoring areas (A1, A2) on either side of a rigid stabilising, shear-resistant central area (C), said two areas each being formed by two tabs (P1, P2), which are symmetrical about a longitudinal axis of the device, capable of being deformed between a closed shape for insertion into a pre-calibrated bone hole, and an open position for anchoring into the bone,
**characterised in that** the tabs, in the open shape thereof, form a positive angle (a1, a2) relative to the base, which is pointed towards the exterior of the base of same, and, in the closed shape thereof, a negative angle (b1, b2) relative to the base, the width at the ends in the closed position (La1f, La2f) being less than the width at the base (L1ab, L2ab).

2. The device according to claim 1, **characterised in that** the tabs (P1, P2) are substantially straight at the base thereof and then rounded inwardly at the end thereof.

3. The device according to claim 2, **characterised in that** the tabs (P1, P2) are substantially straight over 1/3 to one half the length thereof at the base of same, and rounded over 1/3 to one half the length thereof at the end of same.

4. The device according to any of the preceding claims, **characterised in that** the ends of the tabs are capable of nearly touching each other in the closed shape.

5. The device according to any of the preceding claims, **characterised in that** same is made of an implantable shape-memory material having a mechanical elastic or super-elastic property.

6. The device according to any of claims 1 to 4, **characterised in that** same is made of an implantable thermal shape-memory material.

7. The device according to any of the preceding claims, **characterised in that**, in the open shape, the tabs (P1, P2) are arranged so as to form, at the base thereof, an outward angle (a1, a2) relative to the axis of the device of between 5° and 25°, and are curved inwardly towards the end thereof so that the side internally tangent to the end forms an angle of 0° with the adjoining longitudinal axis.

8. The device according to any of the preceding claims, **characterised in that**, in the closed shape, the tabs (P1, P2) form, at the base thereof, an inward angle (b1, b2) of between -0° and -15°, without any significant modification of the latter third of the length thereof, so that the outside width at the ends in the closed shape is less than the width of the base.

9. The device according to any of the preceding claims, **characterised in that** the bases of the anchoring areas (A1, A2) are deformable, in order to enable the tabs (P1, P2) to pass from the closed shape to the open shape.

10. The device according to any of the preceding claims, **characterised in that** the width at the ends (La1, La2) of the open shape is greater than or equal to the width at the ends (La1f, La2f) of the closed shape by 50% or by 1.5 mm.

11. The device according to any of the preceding claims, **characterised in that** the width (La1f, La2f) at the ends of the closed shape is less than the width of the base (L1ab, L2ab) by 20%.

12. The device according to any of the preceding claims, **characterised in that** the ends are bevelled.

13. The device according to any of the preceding claims, **characterised in that** the anchoring areas (A1, A2) have notches (D) on the outer surface of the tabs or a rough surface.

14. The device according to any of the preceding claims, **characterised in that**, in the lower half thereof, same comprises anchoring areas (A1, A2) on the base side, and an area or seat of a specific length defined by notches (D1, D2), for positioning an instrument for closing and holding in closed position.

15. The device according to claim 14, **characterised in that** the central area (C) has a minimum length (Lc) equal to the length (d) of the area or seat between the notches (D1, D2).

16. The device according to any of the preceding claims, **characterised in that** the central area (C) is elbowed and has an angle (Ag) between the two anchoring areas capable of reaching 30°.

17. The device according to any of the preceding claims, **characterised in that** the expansive force of the tabs (P1, P2) between the open shape and the closed shape is between 1 and 3 Kg.

## Patentansprüche

1. Vorrichtung für die intramedulläre Osteosynthese zwischen zwei Knochenteilen, insbesondere der Hand oder des Fußes, bestehend aus einem Körper mit abgeflachtem Hauptquerschnitt und mit zwei Zonen (A1, A2) zur Knochenverankerung beidseitig einer steifen, scherfesten mittigen Stabilitätszone (C), wobei diese zwei Zonen jeweils durch zwei Schenkel (P1, P2) gebildet sind, die symmetrisch um eine Längsachse der Vorrichtung angeordnet sind und zwischen einer geschlossenen Form zur Einführung in ein vorkalibriertes Knochenloch und einer geöffneten Form zur Verankerung im Knochen verformbar sind,
**dadurch gekennzeichnet, dass**
die Schenkel in der geöffneten Stellung an der Basis einen positiven, nach außen in Bezug auf deren Basis weisenden Winkel (a1, a2) und in der geschlossenen Form einen negativen Winkel (b1, b2) an der Basis aufweisen, wobei die Breite an den Enden in der geschlossenen Stellung (La1f, La2f) geringer ist als die Breite an der Basis (L1ab, L2ab).

2. Vorrichtung nach Anspruch 1, **dadurch gekennzeichnet, dass** die Schenkel (P1, P2) an ihrer Basis weitestgehend gerade und anschließen an ihrem Ende nach innen abgerundet sind.

3. Vorrichtung nach Anspruch 2, **dadurch gekennzeichnet, dass** die Schenkel (P1, P2) an ihrer Basis auf 1/3 bis zur Hälfte ihrer Länge weitestgehend gerade und an ihrem Ende auf 1/3 bis zur Hälfte ihrer Länge abgerundet sind.

4. Vorrichtung nach einem beliebigen der vorhergehenden Ansprüche, **dadurch gekennzeichnet, dass** die Enden der Schenkel geeignet sind, sich in der geschlossenen Form nahezu zu berühren.

5. Vorrichtung nach einem beliebigen der vorhergehenden Ansprüche, **dadurch gekennzeichnet, dass** sie aus einem implantierbaren Material mit Formgedächtnis hergestellt ist, das eine mechanisch elastische oder superelastische Eigenschaft aufweist.

6. Vorrichtung nach einem beliebigen der Ansprüche 1 bis 4, **dadurch gekennzeichnet, dass** sie aus einem implantierbaren Material mit thermischem Formgedächtnis hergestellt ist.

7. Vorrichtung nach einem beliebigen der vorhergehenden Ansprüche, **dadurch gekennzeichnet, dass** in der geöffneten Form die Schenkel (P1, P2) so angeordnet sind, dass sie an ihrer Basis mit der Achse der Vorrichtung einen zwischen 5° und 25° liegenden, nach außen weisenden Winkel (a1, a2) bilden, und zu ihrem Ende hin nach innen gekrümmt sind, so dass die innenseitige Tangente an dem Ende mit der Längsachse einen Winkel in der Nähe von 0° bildet.

8. Vorrichtung nach einem beliebigen der vorhergehenden Ansprüche, **dadurch gekennzeichnet, dass** in der geschlossenen Form die Schenkel (P1, P2) an ihrer Basis einen zwischen -0° und -15° liegenden, nach innen weisenden Winkel (b1, b2) ohne signifikante Veränderung auf dem letzte Drittel ihrer Länge bilden, so dass die Außenbreite an den Enden in der geschlossenen Form geringer ist als die Breite der Basis.

9. Vorrichtung nach einem beliebigen der vorhergehenden Ansprüche, **dadurch gekennzeichnet, dass** die Basen der Verankerungszonen (A1, 1, A2) für den Übergang der Schenkel (P1, P2) von der geschlossenen in die geöffnete Form verformbar sind.

10. Vorrichtung nach einem beliebigen der vorhergehenden Ansprüche, **dadurch gekennzeichnet, dass** die Breite an den Enden (La1, La2) der geöffneten Form um 50% bzw. 1,5 mm größer als oder gleich der Breite an den Enden (La1t, La2f) der geschlossenen Form ist.

11. Vorrichtung nach einem beliebigen der vorhergehenden Ansprüche, **dadurch gekennzeichnet, dass** die Breite (La1f, La2f) an den Enden der geschlossenen Form um 20% geringer ist als die Breite der Basis (L1ab, L2ab).

12. Vorrichtung nach einem beliebigen der vorhergehenden Ansprüche, **dadurch gekennzeichnet, dass** die Enden abgeschrägt sind.

13. Vorrichtung nach einem beliebigen der vorhergehenden Ansprüche, **dadurch gekennzeichnet, dass** die Verankerungszonen (A1, A2) Einkerbungen (D) auf der Außenfläche der Schenkel oder eine raue Oberfläche aufweisen.

14. Vorrichtung nach einem beliebigen der vorhergehenden Ansprüche, **dadurch gekennzeichnet, dass** sie in der unteren Hälfte der Verankerungszonen (A1, A2) basisseitig eine Zone bzw. Aufnahme mit einer vorbestimmten Länge aufweist, welche durch Einkerbungen (D1, D2) für die Positionierung eines Instruments zum Schließen und Halten in der geschlossenen Stellung begrenzt ist.

15. Vorrichtung nach Anspruch 14, **dadurch gekennzeichnet, dass** die mittige Zone (C) eine Mindestlänge (Lc) aufweist, welche der Länge (d) der Zone bzw. der Aufnahme zwischen den Kerben (D1, D2) entspricht.

16. Vorrichtung nach einem beliebigen der vorhergehenden Ansprüche, **dadurch gekennzeichnet, dass** die mittige Zone (C) gekrümmt ist und zwischen den beiden Verankerungszonen einen Winkel (Ag) aufweist, der 30° erreichen kann.

17. Vorrichtung nach einem beliebigen der vorhergehenden Ansprüche, **dadurch gekennzeichnet, dass** die Expansionskraft der Schenkel (P1, P2) zwischen der geöffneten Form und der geschlossenen Form zwischen 1 und 3 kg liegt.
